## Europäisches Patentamt

## European Patent Office

## Office européen des brevets

(11) Veröffentlichungsnummer: **0 353 704 B1**

# EUROPÄISCHE PATENTSCHRIFT

(45) Veröffentlichungstag der Patentschrift: **29.11.95**

(51) Int. Cl.⁶: **C11C 3/00**, C14C 9/02, C07C 303/02

(21) Anmeldenummer: **89114168.1**

(22) Anmeldetag: **01.08.89**

Die Akte enthält technische Angaben, die nach dem Eingang der Anmeldung eingereicht wurden und die nicht in dieser Patentschrift enthalten sind.

(54) **Flüssige bzw. fliessfähige Derivate von natürlichen Fetten und Ölen, Verfahren zu ihrer Herstellung und Verwendung.**

(30) Priorität: **02.08.88 DE 3826179**

(43) Veröffentlichungstag der Anmeldung:
**07.02.90 Patentblatt 90/06**

(45) Bekanntmachung des Hinweises auf die Patenterteilung:
**29.11.95 Patentblatt 95/48**

(84) Benannte Vertragsstaaten:
**AT BE CH DE ES FR GB IT LI NL SE**

(56) Entgegenhaltungen:
**EP-A- 0 247 490**
**EP-A- 0 247 509**

**SOVIET INVENTIONS ILLUSTRATED, Sektion D, Woche 46, Zusammenfassung Nr. 304075/46, 27. November 1986, Derwent Publications Ltd., London, GB; & SU-A- 1221252**

**DAS LEDER, Band 39, Nr. 12, Dezember 1988, S. 241, Darmstadt, DE**

(73) Patentinhaber: **Chemische Fabrik Stockhausen GmbH**
**Bäkerpfad 25**
**D-47805 Krefeld (DE)**

(72) Erfinder: **Brehm, Helmut, Dipl.-Ing.**
**Dachsstrasse 22**
**D-4150 Krefeld (DE)**
Erfinder: **Klimmek, Helmut, Dr. Dipl.-Chem.**
**Richard-Strauss-Strasse 32**
**D-4150 Krefeld (DE)**
Erfinder: **Strijbos, Leonhard**
**Sternstrasse 37c**
**D-4154 Tönisvorst (DE)**

(74) Vertreter: **Klöpsch, Gerald, Dr.-Ing. Patentanwalt**
**An Gross St. Martin 6**
**D-50667 Köln (DE)**

EP 0 353 704 B1

**Beschreibung**

Die Erfindung betrifft ein Verfahren zur Herstellung von flüssigen bzw. fließfähigen Derivaten von natürlichen Fetten und Ölen und ihre Verwendung zur Lederfettung, gegebenenfalls in Kombination mit anderen fettend wirkenden Stoffen sowie anionischen und/oder nichtionogenen Emulgatoren zur Fettung von Leder.

In der DE-OS 36 17 657 werden bei Raumtemperatur flüssige Derivate von natürlichen Fetten und Ölen und ein Verfahren zur ihrer Herstellung beschrieben, wobei bei Raumtemperatur feste bzw. feste Anteile enthaltende Fette, deren Gemische mit freien Fettsäuren, Mono- und/oder Diglyceriden bei erhöhten Temperaturen in Gegenwart von basischen Katalysatoren mit wenigstens einem 1,2-Epoxid oxalkyliert und die erhaltenen Umsetzungsprodukte, gegebenenfalls nach Epoxidierung, auf an sich bekannte Weise sulfoniert oder sulfatiert werden. Die so erhaltenen Produkte sind zur Fettung von Leder, insbesondere zur Lickerfettung, geeignet. Mit dem Verfahren nach DE-OS 36 17 657 ist es möglich, in großen Mengen zur Verfügung stehende preisgünstige, feste oder feste Anteile enthaltende Fettrohstoffe, wie Tierkörperfette, in Lederfettungsmittel umzuwandeln.

In der EP-A-247490 wird ein Verfahren zur Herstellung von sulfitierten Fettstoffen beschrieben, in dem Fettstoffe mit Jodzahlen kleiner etwa 100 in Mischung mit Estern von $C_{12}$- bis $C_{24}$-Fettsäuren mit einwertigen $C_1$- bis $C_5$-Alkoholen mit sauerstoffhaltigen Gasgemischen oxidiert und entweder gleichzeitig oder anschließend mit Hydrogensulfiten sulfitiert werden. Hierdurch sollen aus schwer sulfitierbaren Fettstoffen in Wasser gut emulgierbare Produkte mit hoher Emulsionsbeständigkeit und gegenüber hochjodigen Fettstoffen höhere Lichtbeständigkeit erhalten werden.

Natürliche Fette und Öle pflanzlichen und tierischen Ursprungs dienen in erster Linie der menschlichen Ernährung. Immer größere Mengen werden jedoch als nachwachsende Rohstoffe in den verschiedensten Bereichen der Industrie eingesetzt. Hierbei ist die technologische Verwendbarkeit dieser Produkte spezifisch von den jeweiligen Eigenschaften der Fette und Öle abhängig. Diese wiederum werden hauptsächlich durch deren Zusammensetzung und molekularen Aufbau bestimmt. Natürliche Fette und Öle setzen sich im wesentlichen aus Triglyzeriden (Neutralfetten) und - zu einem kleineren Teil - Phosphorlipiden, Mono-, Diglyceriden und freien Fettsäuren zusammen. Die Eigenschaften dieser Substanzgruppe - dies gilt insbesondere für die Neutralfette - wird durch die Art der an das Glyzerinmolekül gebundenen Fettsäuren hinsichtlich der Kettenlänge (kurz-, mittel- und langkettig), durch deren Sättigungsgrad und Konformation (gesättigt, einfach- oder mehrfach ungesättigt; cis-, trans-Anordnung) und durch die Anordnung und Menge pro Glyzerinmolekül bestimmt.

Insgesamt bedeutet dies, daß letztendlich der jeweilige Aufbau der Bausteine der natürlichen Fette und Öle zu einem großen Teil deren technologische Verwendbarkeit bestimmt und sehr häufig auch einschränkt, wenn keine Veränderungen am Molekül vorgenommen werden (können), weil Kostengründe dagegen sprechen oder weil die gewünschte Modifikation konventionell chemisch praktisch nicht herstellbar ist.

Bisherigen Techniken zufolge müssen natürliche Fette und Öle spezifischen Reinigungsschritten bzw. Trennungen in feste und flüssige Phasen oder einer Härtung unterworfen werden. Die gewünschten "Fettchemikalien" entstehen schließlich aus den Spalt- oder Umsetzungsprodukten der natürlichen Öle und Fette: Fettsäuren, Glyzerin und Fettsäuremethylester - die eigentlichen oleochemischen Grundstoffe - und die wegen ihrer Bedeutung für verschiedenste Derivate wichtigen Fettalkohole und Fettamine.

Da der molekulare Aufbau von natürlichen Fetten und Ölen durch deren Herkunft bestimmt wird, Fette und Öle als solche praktisch keine verwendbaren "Fettchemikalien" sind, müssen durch technische Prozesse "maßgeschneiderte" Fette und Öle hergestellt werden. Die hierzu notwendigen Prozesse sind durch hohen Energieverbrauch und hohe Investitionskosten gekennzeichnet. Zudem sind sie häufig wenig spezifisch (Gefahr der Isomerisierung der Fettsäuren, Herstellung von Gemischen statt einheitlichen Produkten usw.).

An Beispielen aus dem Bereich der Lederhilfsmittelindustrie soll deshalb verdeutlicht werden, daß nur bei bestimmter Zusammensetzung der Fettungsmittel eine technologische Brauchbarkeit unter Berücksichtigung von spezifischen Anforderungen gegeben ist:
- Für die technologische Verarbeitbarkeit von Fetten ist es wichtig, daß diese in fließfähiger Form vorliegen. Tierkörperfette, deren Einsatz für die Herstellung von Lederfettungsmitteln wünschenswert wäre, sind fest. Um diese einer Nutzung zugänglich zu machen, müssen sie verflüssigt werden. Dies kann geschehen, indem eine Fraktionierung durchgeführt wird. Dieses Verfahren ist aber aufwendig, mit hohem Energieverbrauch verbunden und auch relativ teuer.
- Auf der Suche nach möglichst billigen, in großer Menge zur Verfügung stehenden Ersatzfetten steht deren technologischen Eignung die Tatsache entgegen, daß es sich hierbei meist um Fette in fester Form handelt, die wiederum erst durch geeignete Maßnahmen verflüssigt werden müssen.

- Fette und Öle mit einer hohen Viskosität erlauben nur eine oberflächliche Fettung von Leder, so daß die Gefahr der Fettfleckigkeit auf dem so behandelten Leder gegeben ist. Ein qualitativ hochwertiges Leder muß durch Fett niedrigerer Viskosität gefettet werden, was wiederum die Einstellung einer bestimmten Viskosität erfordert.

- Für die technologische Weiterverarbeitung von Fetten für Lederfettungsmittel ist es häufig erforderlich, daß Doppelbindungen in den Fettsäuremolekülen vorhanden sind (z.B. zur Durchführung der Sulfonierung). Ausgangsprodukte solcher Art stehen bisher nur in den natürlichen Ölen, die zudem relativ teuer sind, zur Verfügung.

- Auf der anderen Seite sind mehrfach ungesättigte, d.h. entsprechend dünnflüssige Öle für die Lederfettung unerwünscht, weil hier aufgrund des hohen Gehaltes an ungesättigten Doppelbindungen die Gefahr der Verharzung besteht.

Viele Jahrzehnte war das flüssige Produkt Spermöl aus den o.g. technischen Gründen das Mittel der Wahl für die lederverarbeitende Industrie. Spermöl verleiht dem fertigen Leder außergewöhnliche Geschmeidigkeit und wurde seit eh und je für die Produktion von Leder höchster Qualität verwendet. Darüber hinaus konnten die Eigenschaften von Leder niedriger Qualität durch die Behandlung mit Spermöl so verbessert werden, daß es ebenfalls hohen Qualitätsansprüchen genügte.

Als Folge der Bemühungen zum Schutz des Pottwals, dem Lieferanten des Spermöls, vor der Ausrottung wurde die Verwendung von Spermöl in Europa eingestellt. Als Austauschprodukte für Spermöl wurden - speziell in der Lederindustrie - neben Lardöl (der flüssigen Phase des Schweineschmalzes) auch synthetisch hergestelltes Triolein eingesetzt. Die Fettung erfolgt gewöhnlich in Ö/W-Emulsionen mit Hilfe von Lickerölen.

Lickeröle sind selbstemulgierende Produkte, die sich aus einem Neutralöl- und einem Emulgatoranteil zusammensetzen. Dessen Ladungscharakter entsprechend gibt es anionische, kationische, amphotere und nichtionische Fettungsmittel. Häufig wird auch zwischen synthetischen und nativen Lickern unterschieden, wobei die Abgrenzung zwischen beiden immer mehr verschwimmt. Der Emulgatoranteil wird entweder größtenteils im Neutralöl durch z.B. partielle Sulfierung erzeugt oder diesem als separate Komponente beigemischt.

Traditionell erfolgt die Herstellung derartiger Produkte durch Umsetzung von bei Raumtemperatur flüssigen Ölen und Fetten mit Schwefelsäure, Oleum oder $SO_3$. Speziell beim Einsatz von $SO_3$ entstehen oft dunkel gefärbte Produkte. Reine, salzarme Produkte werden durch Sulfierung mit einem $SO_3$-Luftgemisch gewonnen.

Eine der Schwierigkeiten bei der $SO_3$-Sulfierung von bei Raumtemperatur flüssigen Ölen und Fetten besteht darin, daß im Laufe der Reaktion die Viskosität stark ansteigt. Die Reaktionsgeschwindigkeit nimmt ab. Entsprechend länger werden die Reaktionszeiten, was zu Verfärbungen bzw. Verkohlungen führt.

Die $SO_3$-Sulfierung in Fallfirm-Reaktoren wird durch eine Viskositätserhöhung besonders behindert, da die Fließgeschwindigkeit in den dünnen Flüssigkeitsschichten stark abnimmt.

Sulfonierte und sulfitierte native Öle und Fette enthalten $\alpha$-Sulfofettsäuren und Hydroxysulfonate. In synthetischen Lickern findet man Alkan-, $\alpha$-Olefin-, Dialkylbenzol- und Chlorparaffinsulfonate sowie langkettige Fettalkoholsulfonate, Phosphorsäure-, Zitronensäure- und Alkylbernsteinsäureester.

Die emulgierenden, meist polaren Anteile eines Fettungsmittels werden vom Leder vorwiegend in Form von Ionenbindungen oder durch Bildung von stabilen Metallkomplexen in nicht extrahierbarer und nicht migrierbarer Form gebunden.

Die Bindung der emulgierten Anteile erfolgt durch van der Waals'sche Kräfte, über polare Gruppen. Die emulgierenden Anteile beeinflussen die Bindung der emulgierten insofern, als sie für deren Verteilung im Leder verantwortlich sind und durch zwischenmolekulare Kräfte eine Ankerwirkung ausüben.

Die Fettung ist ein qualitätsbestimmender Prozeß der Herstellung von Leder. Diese Feststellung gilt in ganz besonderem Maße für sehr weiche Ledersorten. Folgende Eigenschaften des Leders werden durch die Fettung entscheidend beeinflußt:

1. Weichheit
2. Mechanische Eigenschaften wie Reißfestigkeit, Weiterreißfestigkeit, Dehnung, Narbenelastizität usw.
3. Fülle, Narbenfestigkeiten und Griffeigenschaften
4. Eigenschaften der Lederoberfläche für die nachfolgenden Finish-Prozesse

Es ist bekannt, daß die Weichheit in erster Linie auf einer Trennung der Faserbündel und Fibrillen während der Trocknung beruht. Demnach ist ein wesentliches Kriterium für die weichmachenden Eigenschaften eines Fettungsmittels seine Fähigkeit, die Oberfläche der Fasern und Fibrillen so zu verändern, daß bei der Trocknung Verklebungen nicht auftreten. Ganz wesentlich wird diese Eigenschaft von den emulgierenden Anteilen des Fettungsmittels beeinflußt. Für die elastischen Eigenschaften, wie Zugfestigkeit, Dehnung, Narbenelastiziät usw., spielt die Schmierwirkung der emulgierten Anteile eines Fettlickers eine entscheidende Rolle. Die mit dem Schmiermittel "überzogenen Fasern" haben eine größere Gleitfähigkeit und damit gleichzeitig eine verringerte innere Reibung.

Es ist anzunehmen, daß ein ausgeprägtes Spreitvermögen der emulgierten Anteile ihre Schmierwirkung entscheidend beeinflußt. Zur Erklärung sei darauf hingewiesen, daß unter Spreitvermögen auf einer Oberfläche die Substanzmenge verstanden wird, die erforderlich ist, die Oberfläche mit einer monomolekularen Schicht vollständig zu bedecken. Je größer das Spreitvermögen, desto geringer ist die jeweils erforderliche Substanzmenge. Es fehlen leider bisher noch Untersuchungsergebnisse über den Einfluß des unterschiedlichen Spreitvermögens der emulgierten Anteile eines Fettungsmittels auf den Fettungseffekt. Mit ein Grund dafür ist die aufwendige und komplizierte Meßtechnik. Dazu kommt, daß die emulgierenden Anteile eines Fettlickers das Spreitvermögen der emulgierten Anteile stark beeinflussen können.

Dem Praktiker ist geläufig, daß Menge und Art des Fettungsmittels die Fülle, die Narbenfestigkeit und den Griff des Leders beeinflussen. Was die Füllwirkung betrifft, so beruht ihre Beurteilung fast immer auf subjektiven Beobachtungen. In besonderen Fällen ist die Fülle jedoch durch Messung der Dickenzunahme des Leders objektiv erfaßbar.

Bei dünnen Ledersorten bis zu einer maximalen Dicke von ca. 1,2 mm - für Großviehhäute - wird die Füllwirkung von Fettungsmitteln besonders deutlich. Es ist möglich, bei richtiger Auswahl der Produkte und ggf. durch erhöhten Einsatz die sonst notwendige Menge an Nachgerbstoff zu reduzieren oder auf eine Nachgerbung sogar ganz zu verzichten.

Das Erzielen einer guten Narbenfestigkeit ist bei Weichledersorten über 1,2 mm Stärke oft ein schwierig zu lösendes Problem. Der Hauptgrund für einen "losen Narben" ist der unterschiedliche histologische Aufbau von Narbenschicht - Papillarschicht - einerseits und Retikularschicht andererseits.

Sehr oft aber wird der "lose Narben" durch die falsche Auswahl des Fettungsmittels oder eine ungeeignete Fettungstechnologie verursacht. Zur Vermeidung dieses die Qualität beeinträchtigenden Lederfehlers kommt es darauf an, eine Fettverteilung über den Lederquerschnitt anzustreben, die sicherstellt, daß die mechanischen Eigenschaften, insbesondere die Weichheit der Narben und Retikularschicht, im kritischen Grenzbereich der beiden Schichten annähernd gleich ist.

Der "Griff" des Leders schließlich ist ebenfalls abhängig von Art, Menge und Eigenschaften der verwendeten Fettungsmittel. Er ist objektiv nicht meßbar und auch schwer zu definieren. Die Weichheit und die Festnarbigkeit jedenfalls sind nur Teile dessen, was der Fachmann unter diesem Begriff versteht. Es gibt z.B. einen "runden" Griff oder "festen" Griff, und nur Spezialisten sind in der Lage, den Griff eines Leders sachlich richtig anzugeben..

Die physikalischen Eigenschaften der Lederoberfläche für die nachfolgende Zurichtung werden durch den Aufbau der verwendeten Fettungsmittel entscheidend beeinflußt. Dies gilt vor allem für die bei modernen Zurichtmethoden so wichtige Saugfähigkeit der Lederoberfläche. Es wurde bereits erwähnt, daß die üblichen Lickerfettungsmittel aus einem emulgierenden und einem emulgierten Anteil bestehen. Für das Verhalten der Lederoberfläche für die Nachfolgeprozesse sind vorwiegend die emulgierenden Komponenten verantwortlich. Sie bestimmen den hydrophilen oder hydrophoben Charakter des Leders. Ihr ionisches Verhalten beeinflußt zusätzlich die elektrische Ladung der Oberfläche.

Der vorliegenden Erfindung liegt die Aufgabe zugrunde, unter Verwendung von in großen Mengen zur Verfügung stehenden preisgünstigen, feste oder feste Anteile enthaltenden Fettrohstoffen, wie z.B. Tierkörperfette und Talgfettsäuremethylester als Ausgangsstoffen auf energiesparende Weise Fettderivate herzustellen, die neben der fettenden und gleichzeitig emulgierenden Wirkung auch ein hohes Spreitvermögen besitzen und daher insbesondere als Lederfettungsmittel geeignet sind.

Diese Aufgabe wird bei einem Verfahren zur Herstellung von flüssigen bzw. fließfähigen Derivaten von natürlichen Fetten oder Ölen durch Umsetzung von bei Raumtemperatur festen oder feste Anteile enthaltenden Fetten und/oder deren Gemischen mit freien Fettsäuren und/oder Mono- und/oder Diglyceriden bei erhöhten Temperaturen in Gegenwart basischer Katalysatoren mit wenigstens einem Alkylenoxid, Sulfonierung bzw. Sulfatierung der erhaltenen Umsetzungsprodukte, gegebenenfalls nach Expoxidierung, und gegebenenfalls Neutralisation der Umsetzungsprodukte dadurch gelöst, daß man als Ausgangsmaterial entweder

A) mindestens einen $C_8$- bis $C_{24}$-Fettsäureester eines aliphatischen $C_1$- bis $C_5$-Monoalkohols

oder

B) eine Mischung von mindestens einem $C_8$- bis $C_{24}$-Fettsäureester eines aliphatischen $C_1$- bis $C_5$-Monoalkohols in einem Mischungsanteil von 1 bis 99 Gew.-%, bezogen auf die Gesamtmischung, und den bei Raumtemperatur festen oder feste Anteile enthaltenden Fetten einsetzt, wobei die letztgenannten Ausgangsstoffe gegebenenfalls freie Fettsäuren, Mono- und/oder Diglyceride enthalten.

Die Produkte der Oxalkylierung und gegebenenfalls Epoxidierung führen in der Verfahrensstufe der Sulfierung mit einem $SO_3$-Luftgemisch zu verfahrenstechnischen Vorteilen, wie höherem Durchsatz, besserer Reaktionssteuerung und ergeben hierdurch homogene Produkte mit verbesserter Farbqualität und konstanten Sulfierungsgraden.

Die Oxalkylierung ist eine an sich bekannte Reaktion. Der Mechanismus der Oxalkylierung eines Triglycerids, das praktisch frei von aktiven, d.h. gegenüber Alkylenoxiden reaktionsfähigen Wasserstoffatomen ist, wird in "Tenside 3" (1966, Heft 2, Seite 37) erörtert. In der DE-AS 12 70 542 wird die Umsetzung von bei Raumtemperatur festen und flüssigen Fetten mit Alkylenoxiden mit dem Ziel beschrieben, die oberflächenaktiven Eigenschaften der Fette so zu verändern, daß Waschmittel, Entschäumer, Emulgatoren usw. entstehen.

Überraschenderweise bleibt bei den erfindungsgemäß eingesetzten Ausgangsprodukten nach Sulfitierung bzw. Sulfonierung der fettende Charakter der Oxalkylierungsprodukte nicht nur erhalten, sondern es werden darüber hinaus Eigenschaftsverbesserungen, dieser Produkte bezüglich der Anwendung als Lederhilfsmittel erzielt, wie z.B. erhöhte Lichtechtheit und geringere Wärmevergilbung (siehe Beispiel 22, Tabelle 1).

Die so erhaltenen Produkte zeigen mindestens die gleichen Fettungseigenschaften wie Produkte auf Basis von bei Raumtemperatur flüssigen, hochwertigen Fetten, wie z.B. Klauenöl oder Lardöl.

Durch die der Sulfierung vorgeschalteten Alkoxylierung werden erfindungsgemäß Fettungsmittel erhalten, die völlig einheitliche Emulsionen mit großer Ergiebigkeit liefern, die den herkömmlichen Fettungsmitteln mit zugefügtem Emulgator überlegen sind (die Bezeichnung "Sulfierung" wird hier als gemeinsamer Oberbegriff für die Einführung von Sulfatgruppen und Sulfonsäuregruppen verstanden, die entweder durch Behandlung mit konzentrierter Schwefelsäure oder auch durch oxidierende Sulfitierung in das Fettmolekül eingeführt werden).

Zu den erfindungsgemäß als Ausgangsmaterialien verwendbaren Fetten gehören grundsätzlich alle Triglyceride und deren Abmischungen mit freien Fettsäuren, Mono- und/oder Diglyceriden. Von besonders praktischer Bedeutung ist die Umwandlung von bei Raumtemperatur festen Fetten bzw. Ölen mit einem Trübungspunkt oberhalb dem des Lardöls.

Die verwendbaren Fette können auch teilweise aufgespalten sein, so daß neben Mono- und Diglyceriden auch freie Fettsäure vorliegt. Die SZ der Fette ist nicht kritisch, wie Oxalkylierungsversuche unter Zusatz von freier Fettsäure gezeigt haben.

Als Fettsäureester kommen Produkte auf Basis natürlicher oder synthetischer Fettsäuren der Kettenlänge $C_8$-$C_{24}$ in Frage. Aliphatische Monoalkohole der Kettenlänge $C_1$-$C_5$ dienen als Alkoholkomponente der Fettsäureester. Die Ester können bei Raumtemperatur fest oder flüssig sein. Bei Raumtemperatur flüssige Fettsäureester ergeben niedrigere Viskositäten. Fettsäureester, deren Fettsäurekomponente die Kettenlänge $C_{12}$-$C_{20}$ und deren Alkoholkomponente Methanol, Ethanol, Isopropanol und/ Isobutanol ist, sind bevorzugt. Die Oxalkylierung kann in Gegenwart von geringen Mengen Wasser, wie sie in natürlichen Fetten vorkommen, bzw. durch wässrige Katalysatorlösung mit eingebracht werden können, durchgeführt werden. Beispielhaft werden die folgenden einsatzbaren Epoxide genannt:
Ethylenoxid, Propylenoxid, Butylenoxid, 2-Methyl-2-butenoxid, 3,3-Dimethyl-1-butenoxid, 3,3-Dimethyl-1-butenoxid, $C_6$-$C_{24}$-Epoxide, Styroloxid, 1,2-Epoxibutadien, 1,2-Epoxicyclohexen, sowie Glycidester und -ether. Verwendet man mehr als ein Epoxid, so können diese entweder nacheinander oder als Gemisch mit den Fettstoffen umgesetzt werden.

Als Katalysatoren für die Umsetzung der Alkylenoxide mit den Fetten dienen basische Verbindungen wie Natrium- und Kaliumhydroxid in fester Form oder als wäßrige Lösung, Natriummethylat oder die Alkalisalze von Fettsäuren, wobei Kaliumhydroxid bevorzugt wird.

Die Umsetzung erfolgt nach bekanntem Verfahren bei erhöhter Temperatur. Um eine zügige Abreaktion der Alkylenoxide zu erreichen, hat sich eine Reaktionstemperatur im Bereich von 130 bis 200°C, vorzugsweise 160 bis 180°C, insbesondere 160°C, als zweckmäßig erwiesen.

Je nach Konsistenz der Fette werden 5 bis 100 Gew.-% Alkylenoxid, bevorzugt 10 bis 25 Gew.-% Alkylenoxid, bezogen auf die Fettstoffmenge, angelagert.

Wird die Alkoxilierung mit mehreren 1,2-Epoxiden durchgeführt, so können die Epoxide entweder nacheinander mit den Ausgangsfetten umgesetzt werden, oder die Umsetzung kann mit einem Gemisch der Epoxide durchgeführt werden.

Nach einer Ausführungsform der Erfindung wird die Oxalkylierung nur bei der Fettstoffkomponente durchgeführt, deren Fließpunkt bzw. Trübungspunkt abgesenkt werden soll.

Im Anschluß an die Alkoxilierung Werden die oxalkylierten Fettstoffe bzw. ihre Abmischung mit einem flüssigen, niedrigviskosen Fettstoff nach an sich bekannten Methoden sulfiert. Die Sulfierung kann mit konzentrierter Schwefelsäure bei Raumtemperatur bis leicht erhöhter Temperatur (von ca. 30°C) für einige Stunden vorgenommen werden. Alternativ können Sulfonsäuregruppen durch Behandlung mit Natriumdisulfit in Gegenwart von Luftsauerstoff eingeführt werden. Besonders vorteilhaft ist die kontinuierliche Sulfierung

5

EP 0 353 704 B1

mit einem $SO_3$-Luftgemisch in einem Fallfilmreaktor bei Raumtemperatur.

Im Anschluß an die Sulfierung wird das erhaltene Produkt zweckmäßig mit wässrigem Alkali auf einen pH-Wert in der Nähe des Neutralpunktes (z.B. pH 6,5) eingestellt. Für die Sulfierung können die in der ersten Verfahrensstufe erhaltenen alkoxylierten Fettstoffe oder ihre Abmischungen weiter mit Kohlenwasserstoffen/oder weiteren ungesättigten Fetten oder Fettbestandteilen, wie z.B. Olein, vermischt werden.

Die Sulfierung kann direkt im Anschluß an die Oxalkylierung vorgenommen werden, wobei die Oxalkylierungsprodukte nicht isoliert zu werden brauchen. Nach einer weiteren Ausführungsform der Erfindung werden die oxalkylierten Fette vor der Sulfierung epoxidiert. Dies kann auf bekannte Weise, z.B. mit Wasserstoffperoxid in Gegenwart von Ameisensäure erfolgen.

Vorteilhaft werden die Oxalkylierungsprodukte von leicht flüchtigen Bestandteilen (z.B. durch Destillation, ggf. im Vakuum) befreit.

Der große Vorteil des erfindungsgemäßen Verfahrens besteht darin, daß minderwertige, dunkelgefärbte Fette eingesetzt werden können, die normalerweise durch einen erhöhten Anteil an freien Fettsäuren, z.B. 5 bis 15%, gekennzeichnet sind. Trotzdem gelangt man zu relativ hellfarbigen, geruchsarmen Produkten.

Ein weiterer Vorteil besteht darin, daß Fettsäureester von aliphatischen Monoalkoholen eingesetzt werden können, die bei Raumtemperatur fest sind, beziehungsweise feste Anteile enthalten.

Vorteilhaft werden nach Variante A oder B Fettstoffe mit Jodzahlen unter 100, vorzugsweise unter 70 eingesetzt. Der Fettanteil der Fettstoffmischung weist vorteilhaft eine Jodzahl kleiner als 100, vorzugsweise kleiner als 60 auf, der Anteil der Fettstoffmischung an Fettsäurealkylester besitzt vorteilhaft eine Jodzahl von 0 bis 135.

Das eingesetzte Fettstoffgemisch bei der Variante B kann von 1 bis 99 Gew.-% Fettsäurealkylester, bezogen auf die Gesamtfettstoffmischung, vorzugsweise 20 - 50 Gew.-%, enthalten.

Die Erfindung wird anhand der folgenden Beispiele verdeutlicht:

Verwendete Abkürzungen:

| Abkürzung | Bezeichnung | Bestimmungsmethode |
|---|---|---|
| JZ | Jodzahl | DGF C-V 11 d |
| OHZ | Hydroxylzahl | DGF C-V 17 a |
| SZ | Säurezahl | DGF C-V 2 |
| | Titer | DGF C-IV 3 c |
| VZ | Verseifungszahl | DGF C-V 3 |
| % | Gewichtsprozent | |
| | Steigschmelzpunkt | DGF C-IV 3 a |
| | Trübungspunkt | DGF D-III 3 |
| DGF = Deutsche Gesellschaft für Fettwissenschaft | | |

Trübungspunkt bestimmt nach Abtrennung des bei der Neutralisation angefallenen Salzes im klaren Öl.

Beispiel 1.1. Oxalkylierung

1000 g eines bei 20° C durch feste Anteile pastösen Tierkörperfettes (Trübungspunkt: 22,5° C, JZ: 56) wird mit 100 g Ölsäuremethylester (Titer: - 12° C, VZ: 195, JZ: 88) vermischt und auf 30° C erwärmt. Beim Abkühlen auf 20° C scheidet das klare gelbe Öl weiße Fettanteile aus, die einen festen Bodensatz bilden.

Diese inhomogene Fettmischung wird in einen Druckreaktor gegeben, mit 20 g 45%iger Kalilauge versetzt, sorgfältig mit Stickstoff gespült und auf 160° C aufgeheizt. Bei einer Reaktionstemperatur von 160 - 165° C werden insgesamt 354 g Propylenoxid dosiert, so daß ein Druck von 6 bar nicht überschritten wird. Die Reaktion ist nach 3 Stunden, inkl. einer Nachreaktionszeit von 1 Stunde, beendet. Nach dem Abkühlen auf ca. 30° C neutralisiert man mit 96%iger Schwefelsäure. Gewonnen wird ein bei 20° C klares, gelbes Öl.
Viskosität bei 20° C: 35 mPa.s (n. Brookfield RV 1/10 Upm)

| | |
|---|---|
| OHZ: | 43,7 |
| VZ: | 168,4 |
| JZ: | 58,2 |
| SZ: | 3,1 |
| Trübungspunkt | 4,3° C |

Beispiel 1.2. Sulfonierung/Sulfatierung (in Gegenwart von Kohlenwasserstoffen und ungesättigten Fettsäuren)

560 g Oxalkylat aus Beispiel 1.1 werden mit 240 g eines $C_{10}$-$C_{30}$-Kohlenwasserstoffgemisches und 200 g Tallölfettsäure vermischt und unter kräftigem Rühren bei 30 - 32 °C in 5 Stunden mit 300 g 96%iger Schwefelsäure umgesetzt. Nach einer Nachreaktionszeit von 1 Stunde neutralisiert man mit 30%iger Natronlauge und verrührt mit 1000 g 20%iger Kochsalzlösung. Nach erfolgter Phasentrennung wird die Salzlösung abgezogen, der pH-Wert auf 6,5 und der Wassergehalt auf 20 Gew.-% eingestellt.

| | |
|---|---|
| org. gebundenes $SO_3$: | 5,4 Gew.-% |
| Steigschmelzpunkt: | 3,5 °C |

Beispiel 1.3. Sulfonierung/Sulfatierung (in Abwesenheit von Kohlenwasserstoffen und ungesättigten Fettsäuren)

1000 g Oxalkylat aus Beispiel 1.1 werden in Abwesenheit von Kohlenwasserstoffen unter kräftigem Rühren bei 30 - 32 °C in 5 Stunden mit 300 g 96%iger Schwefelsäure umgesetzt. Nach einer Nachreaktionszeit von 1 Stunde wird wie in Beispiel 1.2 die Neutralisation und Aufarbeitung durchgeführt. Das bei 20 °C klare Öl hat einen Gehalt an organisch gebundenem $SO_3$ von 5,3 Gew.-%.

Beispiel 1.4. Oxydierende Sulfitierung

Eine Mischung von 700 g Reaktionsprodukt aus Beispiel 1.1 und 300 g eines Kohlenwasserstoffgemisches der Kettenlänge $C_{10}$-$C_{22}$ wird bei 90 - 110 °C unter Durchleiten von Luft oxidiert, bis die Jodzahlabnahme 20 beträgt. Das Oxidat sulfitiert man bei 80° C durch Zugabe von 9 Gew.-% Natriumdisulfit in Form einer bei 50° C gesättigten Lösung. Das auf pH 6,7 eingestellte Produkt ist ein bei 20° C opales Öl.

Beispiel 2.1. Oxalkylierung

Eine bei 20° C feste Anteile enthaltende Mischung der Rohstoffe aus Beispiel 1.1, bestehend aus 1600 g Tierkörperfett und 400 g Ölsäuremethylester, wird wie in Beispiel 1.1 mit Propylenoxid umgesetzt. Gewonnen wird ein bei 20° C dünnflüssiges, opales gelbes Öl.

| | |
|---|---|
| OHZ: | 50,9 |
| VZ: | 172,5 |
| JZ: | 55,5 |
| SZ: | 4,0 |
| Trübungspunkt | 4,4° C |

Beispiel 2.2. Sulfonierung/Sulfatierung

Das Oxalkylierungsprodukt wird auf die gleiche Weise wie in Beispiel 1.2 sulfoniert/sulfatiert, wobei ein Produkt mit einem Gehalt an organisch gebundenem $SO_3$ von 5,4 Gew.-% und einem Steigschmelzpunkt von 6,0° C erhalten wird.

Beispiel 3.1. Oxalkylierung

1000 g Tierkörperfett aus Beispiel 1.1 und 1000 g Stearinsäuremethylester (Titer: ca. 27° C, VZ: 196) werden bei 160° C in Gegenwart von 20 g 45%iger Kalilauge mit 865 g Propylenoxid umgesetzt. Nach der Neutralisation mit 96%iger Schwefelsäure erhält man ein schwach trübes, gelbes Öl.

| Trübungspunkt: | 12,5° C |
|---|---|
| Viskosität 20° C: | 70 mPa.s (n. Brookfield Sp. 1/10 Upm) |
| OHZ: | 43,0 |
| VZ: | 141,4 |
| JZ: | 23,8 |
| SZ: | 3,0 |

Beispiel 3.2. Sulfonierung/Sulfatierung

Das Oxalkylierungsprodukt wird auf die gleiche Weise wie in Beispiel 1.2 sulfoniert/sulfatiert. Es wird ein Reaktionsprodukt mit einem Gehalt an organisch gebundenem $SO_3$ von 3,9 Gew.-% und einem Steigschmelzpunkt von 10,5° C erhalten.

Beispiel 4.1 Oxalkylierung

Eine Mischung aus 600 g Rindertalg (VZ: 198, JZ: 42, Titer: 36,2° C) und 1400 g Ölsäuremethylester (VZ: 190, JZ: 105) werden wie in Beispiel 1.1. mit Propylenoxid umgesetzt.

| Trübungspunkt: | - 0,6° C |
|---|---|
| Viskosität bei 20° C | 32 mPa.s (n. Brookfield Sp. 1/10 Upm) |
| OHZ: | 45,5 |
| VZ: | 162,0 |
| JZ: | 59,0 |
| SZ: | 3,2 |

Beispiel 4.2 Sulfonierung/Sulfatierung

Sulfonierung/Sulfatierung werden auf die gleiche Weise wie in Beispiel 1.2 durchgeführt, wobei ein Produkt mit einem Gehalt an organische gebundenem $SO_3$ von 5,2 Gew.-% und einem Steigschmelzpunkt von 5,0° C erhalten wird.

Beispiel 5.1. Oxalkylierung

Eine Mischung aus 1400 g Rindertalg und 600 g Ölsäuremethylester, Rohstoffe wie sie in Beispiel 4 verwendet wurden, wird wie in Beispiel 1.1 mit Propylenoxid umgesetzt.

| Trübungspunkt: | 10,4° C |
|---|---|
| Viskosität bei 20° C: | 52 mPa.s (n. Brookfield Sp. 1/10 Upm) |
| OHZ: | 51,0 |
| VZ: | 171,3 |
| JZ: | 51,5 |
| SZ: | 3,60 |

Beispiel 5.2. Sulfonierung/Sulfatierung

Das Oxalkylierungsprodukt wird auf die gleiche Weise wie in Beispiel 1.2 sulfoniert/sulfatiert. Es wird ein Reaktionsprodukt mit einem Gehalt an organisch gebundenem $SO_3$ von 5,5 Gew.-% und einem Steigschmelzpunkt von 14,0° C erhalten.

Zur erfindungsgemäßen Verwendung:

Beispiele 6 - 10

Chromgegerbte, mit pflanzlichem, synthetischem und Harzgerbstoff nachgegerbte, gefärbte Schuhoberleder von ca. 2 mm Falzstärke aus Rindhäuten werden bei 40° C 45 Minuten mit 100 % Flotte und 7 % der in Beispiel 1.2 bis 5.2 gewonnenen Produkte gelickert. Die Leder werden in üblicher Weise getrocknet und fertiggestellt. Man erhält sehr weiche Leder mit hoher Festnarbigkeit und Farbegalität.

Beispiel 11

Chromgegerbte, mit anionischem Polymergerbstoff nachgegerbte, gefärbte Rinderhäute, Falzstärke bis 0,8 bis 1,0 mm, werden bei 50° C in 150 % Flotte während 60 Minuten mit 10 % des nach Beispiel 1.3 erhaltenen Produkts (bezogen auf das Falzgewicht) gelickert. Nach praxisüblicher Trocknung und Fertigstellung erhält man sehr weiche, geschmeidige Bekleidungs- und Möbelleder mit sehr egalem Millkorn und hoher Lichtbeständigkeit.

Beispiel 12

Die in der Tabelle 1 aufgeführten oxalkylierten Fettstoffe und ihre Sulfonierungs- bzw. Sulfatierungsprodukte werden im Vergleich zu nicht oxalkylierten flüssigen sulfonierten bzw. sulfatierten Fettstoffen auf Lichtechtheit und Wärmevergilbung geprüft. Die Lichtechtheitsprüfung erfolgt nach DIN 54004. Dazu werden die aufgelisteten Stoffe mit 20 Gew.-%, bezogen auf Papiergewicht, auf Chromatographiepapier Nr. 2040a (Fa. Schleicher[R] & Schüll) aufgetragen und 72 Stunden belichtet. Die Beurteilung erfolgt anhand der 8-stufigen Baumwollskala. Je höher die Lichtbeständigkeit der Öle ist, umso höher ist die Stufennummer. Zur Prüfung der Wärmevergilbung wird eine Probe wie oben beschrieben vorbereitet und 24 Stunden bei 100° C gelagert. Die Beurteilung der Vergilbung erfolgt nach DIN 54004.

## Tabelle 1:

| Produkt | Lichtechtheit | Wärmevergilbung |
|---|---|---|
| Zeit(h) | 72 | 24 |
| Capelinöl* | 2 - 3 | 1 |
| Lardöl | 4 | 2 - 3 |
| Beisp.1.3. | 4 | 4 |

* Capelinöl ist ein spezielles Fischöl

Beispiel 13: Emulgierverhalten

Zur Überprüfung des Emulgierverhaltens werden die Oxalkylate aus Beispiel 1.1. und 2.1. sowie zum Vergleich Lardöl nach Beispiel 1.3. sulfoniert bzw. sulfatiert und mit einem Mineralöl vermischt. Die Emulsionsstabilität der Mischungen nach Zugabe von 60°C warmem Leitungswasser im Verhältnis 1 : 4 wird beurteilt.

Tabelle 2:

| Sulfonat nach Beispiel 1.3. aus | Mischung | | Beurteilung der Emulsion |
|---|---|---|---|
| | Sulfonat Gew.-% | Mineralöl* Gew.-% | nach 1 Stunde |
| Lardöl | 20 | 80 | nicht stabil, 2 Phasen |
| Beispiel 1.1. | 20 | 80 | stabil |
| Beispiel 2.1. | 20 | 80 | stabil |

\* Siedebereich: 200 - 350° C/0,266 bar

Viskosität 20° C: 65 cPs

Dichte 20° C: 0,893 g/cm$^3$

Beispiel 14:

Beispiel 2.1 wird wiederholt, nur daß anstelle von Ölsäuremethylester
a) Ölsäureisopropylester
b) Ölsäurebutylester
eingesetzt werden.

800 g der erhaltenen, bei Raumtemperatur flüssigen Oxalkylate, werden jeweils mit 200 g Tallölfettsäure vermischt und mit 300 g 96%iger Schwefelsäure wie in Beispiel 1.2 sulfatiert und aufgearbeitet.

Tabelle 3

| Produkt nach Beispiel | organ. geb. SO$_3$ % | Steigschmelzpunkt ° C | Lichtechtheit nach 72 Std. | Wärmevergilbung 100 ° C/24 Std. |
|---|---|---|---|---|
| 14a | 5,3 | 12,7 | 4 | 3 |
| 14b | 5,2 | 12,5 | 4 | 3 |

**Patentansprüche**

1. Verfahren zur Herstellung von flüssigen bzw. fließfähigen Derivaten von C$_8$-C$_{24}$-Fettsäureestern von aliphatischen C$_1$-C$_5$-Monoalkoholen oder Mischungen dieser Fettsäureester mit natürlichen Fetten oder Ölen, welche bei Raumtemperatur fest sind oder feste Anteile enthalten und/oder deren Gemischen mit freien Fettsäuren und/oder Mono- und/oder Diglyceriden durch Umsetzung mit wenigstens einem Alkylenoxid bei erhöhten Temperaturen in Gegenwart basischer Katalysatoren, Sulfonierung bzw. Sulfatierung der erhaltenen Umsetzungsprodukte, gegebenenfalls nach Epoxidierung und gegebenenfalls Neutralisation der Umsetzungsprodukte, dadurch gekennzeichnet, daß man als Ausgangsstoff entweder

A) mindestens einen $C_8$-$C_{24}$-Fettsäureester eines aliphatischen $C_1$-$C_5$-Monoalkohols

oder

B) eine Mischung von mindestens einem $C_8$-$C_{24}$-Fettsäureester eines aliphatischen $C_1$-$C_5$-Monoalkohols in einem Mischungsanteil von 1 bis 99 Gew.-%, bezogen auf die Gesamtmischung, und den bei Raumtemperatur festen oder feste Anteile enthaltenden Fetten einsetzt, wobei die letztgenannten Ausgangsstoffe gegebenenfalls freie Fettsäuren, Mono- und/oder Diglyceride enthalten.

2. Verfahren nach Anspruch 1, dadurch gekennzeichnet, daß man als Ausgangsstoff eine Mischung einsetzt, die 20 bis 50 Gew.-% $C_{18}$-$C_{24}$-Fettsäureester eines aliphatischen $C_1$-$C_5$-Monoalkohols enthält.

3. Verfahren nach einem der Ansprüche 1 bis 2, dadurch gekennzeichnet, daß man mit 5 bis 100 Gew.-% Alkylenoxid, vorzugsweise 10 bis 25 Gew.-% Alkylenoxid, bezogen auf die Menge des eingesetzten Ausgangsstoffs, oxalkyliert.

4. Verfahren nach einem der Ansprüche 1 bis 3, dadurch gekennzeichnet, daß zur Oxalkylierung eine oder mehrere Alkylenoxide eingesetzt werden, wobei bei Verwendung von mehr als einem Alkylenoxid die Alkylenoxide entweder nacheinander oder im Gemisch eingesetzt werden.

5. Verfahren nach einem der Ansprüche 1 bis 4, dadurch gekennzeichnet, daß die Oxalkylierung in Gegenwart basischer Katalysatoren, wie Natrium- oder Kaliumhydroxid oder -alkoholat oder Alkalisalzen von Fettsäuren, durchgeführt wird.

6. Verfahren nach einem der Ansprüche 1 bis 5, dadurch gekennzeichnet, daß die Oxalkylierung bei Temperaturen vo 130 bis 200° C, vorzugsweise 160 bis 180° C, durchgeführt wird.

7. Verfahren nach einem der Ansprüche 1 bis 6, dadurch gekennzeichnet, daß man Ausgangsstoffe mit Jodzahlen unter 100, bevorzugt unter 70, einsetzt.

8. Verfahren nach einem der Ansprüche 1 bis 7, dadurch gekennzeichnet, daß als Fettsäureester die Methyl-, Ethyl-Isopropyl- und/oder Isobutylester eingesetzt werden.

9. Verfahren nach einem der Ansprüche 1 bis 8, dadurch gekennzeichnet, daß die Sulfierung der Oxalkylierungsprodukte im Gemisch mit Kohlenwasserstoffen und/oder ungesättigten Fettsäuren durchgeführt wird und die erhaltenen Reaktionsprodukte neutralisiert werden.

10. Flüssige bzw. fließfähige Derivate von natürlichen Fetten und Ölen, erhältlich nach einem Verfahren gemäß den Ansprüchen 1 bis 9, gekennzeichnet durch eine Lichtechtheit, gemessen nach 72 Stunden, von mindestens 4 und eine Wärmevergilbung, gemessen nach 24 Stunden, von mindestens 3, bestimmt nach DIN 54004.

11. Verwendung der flüssigen bzw. fließfähigen Derivate von natürlichen Fetten und Ölen gemäß Anspruch 10 bzw. des Verfahrensproduktes gemäß Ansprüchen 1 bis 9, gegebenenfalls in Kombination mit anderen fettend wirkenden Stoffen sowie anionischen und/oder nichtionogenen Emulgatoren zur Fettung von Leder.

**Claims**

1. A process for the production of liquid or flowable derivatives of $C_8$-$C_{24}$ fatty acid esters of aliphatic $C_1$-$C_5$ monoalcohols or mixtures of these fatty acid esters with natural fats or oils that are solid at room temperature or comprise solid fractions and/or their mixtures with free fatty acids and/or mono- and/or diglycerides by reaction with at least one alkylene oxide at elevated temperatures in the presence of alkaline catalysts, sulfonation or sulfation of the obtained reaction products, optionally after epoxidation,

EP 0 353 704 B1

and optional neutralization of the reaction products, characterized in that as starting material either

A) at least one $C_8$-$C_{24}$ fatty acid ester of an aliphatic $C_1$-$C_5$ monoalcohol

or

B) a mixture of at least one $C_8$-$C_{24}$ fatty acid ester of an aliphatic $C_1$-$C_5$ monoalcohol in a mixture ratio of 1 to 99%-wt., relative to the total mixture, and the fats that are solid at room temperature or comprise solid fractions are used, the latter starting materials optionally comprising free fatty acids, mono- and/or diglycerides.

2. The process according to claim 1 characterized in that a mixture comprising 20 to 50%-wt. of $C_8$-$C_{24}$ fatty acid ester of an aliphatic $C_1$-$C_5$ monoalcohol is used as starting material.

3. The process according to any one of claims 1 to 2 characterized in that oxalkylation is carried out with 5 to 100%-wt. of alkylene oxide, preferably 10 to 25%-wt. of alkylene oxide, relative to the amount of the starting material used.

4. The process according to any one of claims 1 to 3 characterized in that one or several alkylene oxides are used for the oxalkylation, and in case more than one alkylene oxide is used, the alkylene oxides are added either successively or as a mixture.

5. The process according to any one of claims 1 to 4 characterized in that the oxalkylation is carried out in the presence of alkaline catalysts, such as sodium or potassium hydroxide or alcoholate or alkali salts of fatty acids.

6. The process according to any one of claims 1 to 5 characterized in that the oxalkylation is carried out at temperatures ranging from 130 to 200°C, preferably 160 to 180°C.

7. The process according to any one of claims 1 to 6 characterized in that starting materials having iodine numbers of below 100, preferably below 70, are used.

8. The process according to any one of claims 1 to 7 characterized in that the methyl, ethyl, isopropyl, and/or isobutyl esters are used as fatty acid esters.

9. The process according to any one of claims 1 to 8 characterized in that the sulfation of the oxalkylation products is carried out in admixture with hydrocarbons and/or unsaturated fatty acids, and that the resulting reaction products are neutralized.

10. Liquid or flowable derivatives of natural fats and oils obtainable according to a process as defined in claims 1 to 9, characterized by a light fastness of at least 4, measured after 72 hours, and a heat-yellowing value of at least 3, measured after 24 hours, determined following the method of DIN 54004.

11. The use of the liquid or flowable derivatives of natural fats and oils according to claim 10 or of the product obtained according to the process of claims 1 to 9, optionally in combination with other substances having lubricating properties and anionic and/or non-ionic emulsifiers for the fat-liquoring and greasing of leather.

**Revendications**

1. Procédé de préparation de dérivés liquides ou fluides d'esters d'acides gras en $C_8$ à $C_{24}$ de monoalcools aliphatiques en $C_1$ à $C_5$, ou de mélanges de ces esters d'acides gras et d'huiles ou graisses naturelles, qui, à la température ambiante, sont solides, ou contiennent des fractions solides et/ou leurs mélanges à des mono- et/ou diglycérides et/ou des acides gras libres, par réaction avec au moins un oxyde d'alkylène, à des températures élevées et en présence de catalyseurs basiques, sulfonation ou sulfatation des produits de réaction obtenus, éventuellement après époxydation et éventuellement neutralisation des produits de réaction, caractérisé en ce que, à titre de matière de départ, on met en oeuvre, soit

A) au moins un ester d'acide gras en $C_8$ à $C_{24}$ d'un monoalcool en $C_1$ à $C_5$,

soit

12

B) un mélange d'au moins un ester d'acide gras en $C_8$ à $C_{24}$ d'un monoalcool aliphatique en $C_1$ à $C_5$ en une proportion de mélange de 1 à 99% en poids, par rapport au mélange global et de graisses solides à la température ambiante, ou contenant des fractions solides à la température ambiante, où les matières de départ citées en dernier lieu contiennent éventuellement des mono- et/ou diglycérides et/ou des acides gras, libres.

2. Procédé suivant la revendication 1, caractérisé en ce que l'on utilise, à titre de matière de départ, un mélange qui contient 20 à 50% en poids d'un ester d' acide gras en $C_{18}$ à $C_{24}$ d'un monoalcool aliphatique en $C_1$ à $C_5$.

3. Procédé suivant l'une quelconque des revendications 1 et 2, caractérisé en ce que l'on procède à l'oxalkylation avec 5 à 100% en poids d'oxyde d'alkylène, de préférence, 10 à 25% en poids d'oxyde d'alkylène, par rapport à la quantité de la matière de départ mise en oeuvre.

4. Procédé suivant l'une quelconque des revendications 1 à 3, caractérisé en ce que, en vue de l'oxalkylation, on met en oeuvre un ou plusieurs oxydes d'alkylène, où, lors de l'utilisation de plus d'un oxyde d'alkylène, on utilise les oxydes d'alkylène les uns après les autres ou en mélange.

5. Procédé suivant l'une quelconque des revendications 1 à 4, caractérisé en ce que l'on entreprend l'oxalkylation en présence de catalyseurs basiques, comme un alcoolate ou l'hydroxyde de sodium ou de potassium, ou de sels de métaux alcalins d'acides gras.

6. Procédé suivant l'une quelconque des revendications 1 à 5, caractérisé en ce que l'on entreprend l'oxalkylation à des températures de 130 à 200°C, de préférence 160 à 180°C.

7. Procédé suivant l'une quelconque des revendications 1 à 6, caractérisé en ce que l'on utilise des matières de départ avec des indices d'iode inférieurs à 100, de préférence inférieurs à 70.

8. Procédé suivant l'une quelconque des revendications 1 à 7, caractérisé en ce que l'on utilise les esters méthyliques, éthyliques, isopropyliques et/ou isobutyliques à titre d'esters d'acides gras.

9. Procédé suivant l'une quelconque des revendications 1 à 8, caractérisé en ce que l'on entreprend la sulfuration des produits de l'oxalkylation en mélange à des hydrocarbures et/ou des acides gras insaturés et on neutralise les produits de réaction obtenus.

10. Dérivés liquides ou fluides de graisses et d'huiles naturelles, que l'on obtient par mise en oeuvre du procédé suivant l'une quelconque des revendications 1 à 9, caractérisés par une solidité à la lumière, mesurée après 72 heures, d'au moins 4 et un jaunissement thermique, mesuré après 24 heures, d'au moins 3, selon la norme DIN 54004.

11. Utilisation des dérivés liquides ou fluides d'huiles et de graisses naturelles selon la revendication 10 ou du produit obtenu par mise en oeuvre du procédé suivant l'une quelconque des revendications 1 à 9, éventuellement en combinaison avec d'autres substances nourrissantes, comme aussi d'émulsifs anioniques et/ou non ioniques, en vue de la nourriture du cuir.